# FASCICULE DE BREVET EUROPEEN

(11) **EP 3 529 777 B1**
(45) Date de publication et mention de la délivrance du brevet: **04.11.2020**
(21) Numéro de dépôt: 17780487.9
(22) Date de dépôt: 18.09.2017
(51) Int. Cl.: G06T 7/10, A61B 34/10, A61B 90/00

(54) **PROCÈDE D'ASSISTANCE A LA RÉALISATION D'UN DISPOSITIF SUR MESURE DÉPLOYABLE IMPLANTABLE**
VERFAHREN ZUR UNTERSTÜTZUNG DER HERSTELLUNG EINER IMPLANTIERBAREN ENTFALTBAREN MESSENDEN VORRICHTUNG
METHOD OF ASSISTING THE PRODUCTION OF AN IMPLANTABLE UNFURLABLE MADE TO MEASURE DEVICE

(30) Priorité: 18.10.2016 FR 1660082
(43) Date de publication de la demande: 28.08.2019
(73) Titulaire: Institut Mines-Télécom, 75014 Paris (FR); Université Jean Monnet Saint Etienne, 42100 Saint-Etienne (FR); Centre Hospitalier Universitaire de Saint-Etienne, 42100 Saint-Etienne (FR)
(72) Inventeur: PERRIN, David, 42000 Saint-Etienne (FR); AVRIL, Stéphane, 42000 Saint-Etienne (FR); BADEL, Pierre, 42580 La Tour en Jarez (FR); ALBERTINI, Jean-Noël, 42290 Sorbiers (FR)
(74) Mandataire: Novagraaf Technologies
(86) Numéro de dépôt international: PCT/FR2017/052486
(87) Numéro de publication internationale: WO 2018/073505

(56) Documents cités:
- WO-A1-2013/171039
- WO-A1-2015/089118
- WO-A1-2015/101545

## Description

L'invention concerne un procédé d'assistance à la réalisation d'un dispositif sur mesure déployable implantable dans une cavité d'un corps d'un être vivant. En particulier, le procédé selon l'invention se base sur une série d'images radiographiques de la cavité.

Actuellement, dans l'optique de réaliser une endoprothèse vasculaire sur mesure adaptée à un patient donné, de simples mesures géométriques du segment d'artère cible sont réalisées sur des images d'un scanner préopératoire du patient. Ces mesures comprennent la distance entre les ostias des artères collatérales débouchant dans le segment cible, leur angulation, etc... Cependant, la difficulté de réalisation des mesures tridimensionnelles, d'une part, et, d'autre part, la difficulté à anticiper des déformations des endoprothèses chez le patient nécessitent en conséquence énormément d'expérience et de temps, à la fois au praticien qui va réaliser l'intervention d'implantation et aux attachés cliniques des fabricants d'endoprothèses pour la réalisation de l'endoprothèse sur mesure. En outre, les incertitudes quant à l'exactitude du dimensionnement des endoprothèses sur mesure conduisent certains fabricants d'endoprothèses à valider le dimensionnement relevé au moyen d'une implantation *in vitro* d'un prototype d'endoprothèse dans un modèle du segment d'artère cible du patient obtenu par impression tridimensionnelle. En pratique, une telle validation *in vitro* nécessite la création de plusieurs prototypes. Par conséquent, cela entraîne un fort surcoût de ces endoprothèses par rapport aux endoprothèses génériques et un grand délai de livraison rédhibitoire pour les patients devant être opérés rapidement. Le document US2013/0296998 décrit un exemple de réalisation d'un modèle de segment d'artère cible par impression tridimensionnelle pour la réalisation d'une endoprothèse sur mesure.

Un but de l'invention est de fournir un procédé d'assistance à la réalisation d'un dispositif sur mesure déployable implantable dans une cavité d'un corps d'un être vivant qui soit simple, rapide et économique.

A cette fin, il est prévu, selon l'invention, un procédé d'assistance à la réalisation d'un dispositif sur mesure déployable implantable dans une cavité d'un corps d'un être vivant à partir d'une série d'images radiographiques de la cavité, le procédé comportant des étapes de :
a. Reconstruction tridimensionnelle d'une surface complexe délimitant une partie de la cavité dans laquelle le dispositif sur mesure déployable est destiné à être mis en place ;
b. Réalisation d'un premier maillage par éléments finis d'une géométrie de la surface complexe ainsi reconstruite ;
c. Choix d'un ou plusieurs composants génériques du dispositif sur mesure déployable dont la personnalisation est à réaliser ;
d. Réalisation d'un deuxième maillage par éléments finis du ou des composants génériques choisis et assemblés pour former le dispositif sur mesure déployable ;
e. Détermination des paramètres d'une morphose du premier maillage obtenu vers un maillage d'une géométrie formée d'un ensemble de volumes simples raccordés les uns aux autres représentatif de la partie de la cavité, puis déformation du premier maillage par cette morphose;
f. Simulation par la méthode des éléments finis de la déformation du deuxième maillage ainsi obtenu, par morphose inverse en fonction des paramètres de la morphose précédemment déterminés, vers la géométrie de la surface complexe de la cavité de sorte à simuler un déploiement du dispositif sur mesure déployable une fois mis en place dans la cavité ;
g. Détermination d'un dimensionnement de points d'intérêt de la partie considérée de la cavité par projection de ceux-ci sur le deuxième maillage ainsi déformé ; et
h. Génération d'un plan coté du ou des composants génériques sur lesquels le dimensionnement précédemment déterminé a été transposé, le plan coté permettant la réalisation du dispositif sur mesure déployable.

Ainsi le procédé d'assistance à la réalisation d'un dispositif sur mesure déployable implantable dans une cavité d'un corps d'un être vivant selon l'invention permet donc de dimensionner numériquement des dispositifs implantables de manière personnalisée au patient à opérer, en phase de planning préopératoire, avant l'intervention chirurgicale d'implantation chez le patient, et ce de manière simple et rapide, tout en restant économique.

Avantageusement, mais facultativement, le procédé selon l'invention comporte au moins l'une des caractéristiques techniques supplémentaires suivantes :
- lors de l'étape b, le procédé comporte une sous étape de détermination de paramètres géométriques de points d'intérêt de la partie considérée de la cavité, les paramètres géométriques étant utilisés lors de l'étape g ;
- suite à l'étape e et préalablement à l'étape f, le deuxième maillage subit une déformation simulant une compression du dispositif sur mesure déployable assemblé à l'intérieur d'une gaine d'implantation ;
- lors de l'étape f, le procédé comporte une étape préliminaire d'insertion du deuxième maillage dans le premier maillage déformé ;
- suite à l'étape f et préalablement à l'étape g, le procédé comporte une étape de calcul par simulation par la méthode des éléments finis de déformations et contraintes induites par des interactions entre les premier et deuxième maillages suite à la déformation par morphose inverse du deuxième maillage vers le premier maillage ;
- lors de l'étape g, la projection consiste à déterminer les nœuds du deuxième maillage en regard des points d'intérêt ;
- lors de l'étape h, la transposition du dimensionnement s'effectue à partir des coordonnées des nœuds précédemment déterminés lors de l'étape g dans le deuxième maillage non déformé ;
- suite à la déformation simulant une compression, le deuxième maillage ainsi déformé subit une simulation d'introduction dans le premier maillage selon une position proche d'une position optimale ;
- le procédé comporte une étape supplémentaire de
   i- Extraction d'un ensemble d'indicateurs permettant à un praticien de déterminer la pertinence d'un planning préopératoire ;
- la cavité étant un lumen artériel, le dispositif sur mesure déployable est une endoprothèse aortique.

D'autre part, il est aussi prévu, selon l'invention, un support informatique comportant l'enregistrement d'un programme de commande d'un calculateur, pour la mise en œuvre du procédé selon l'invention présentant au moins l'une des caractéristiques précédentes.

D'autres caractéristiques et avantages de l'invention apparaîtront lors de la description ci-après d'un mode de réalisation du procédé. Aux dessins annexés :
- La figure la est une vue tridimensionnelle d'un maillage par éléments finis d'un segment d'artère cible ;
- La figure 1b est une vue tridimensionnelle d'une morphose du segment d'artère cible de la figure la par le procédé selon l'invention ;
- La figure 2a est une vue de face d'un maillage par éléments finis d'une endoprothèse sur mesure en cours d'élaboration ;
- la figure 2b est une vue de face du maillage de la figure 2a dans une configuration comprimée à l'intérieur d'une gaine pour implantation ;
- la figure 3 est une vue illustrant la morphose du maillage de la figure 2b implanté dans le segment d'artère cible morphosé de la figure 1b vers le segment d'artère cible de la figure la, par le procédé selon l'invention ;
- la figure 4 est une vue schématique illustrant le dimensionnement de l'endoprothèse sur mesure et le positionnement des fenêtres sur le maillage de l'endoprothèse de la figure 2a, par le procédé selon l'invention ; et,

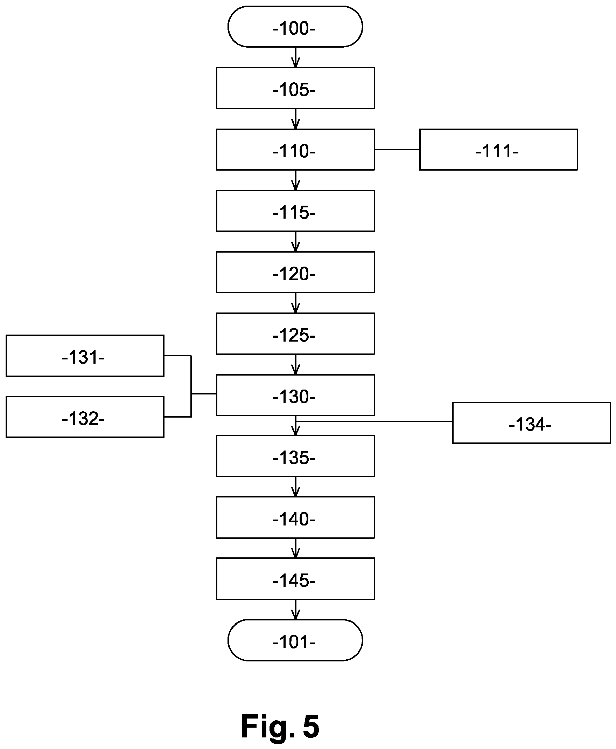

- la figure 5 est un organigramme du procédé selon l'invention.

En référence aux figures, nous allons décrire plus en détails un procédé 100 d'assistance à la réalisation d'un dispositif sur mesure déployable implantable dans une cavité d'un corps d'un être vivant selon l'invention.

Le procédé 100 d'assistance à la réalisation d'un dispositif sur mesure déployable implantable dans une cavité d'un corps d'un être vivant selon l'invention comporte une première étape 105 durant laquelle une reconstruction tridimensionnelle d'un segment d'artère cible 1 est réalisée. Cette reconstruction 105 s'effectue à partir d'une série d'images radiographiques préopératoires du segment d'artère cible 1 du patient à opérer. Ici, le segment d'artère cible 1, aussi appelé lumen artériel, forme une cavité du corps d'un être vivant qu'est le patient, au sens de l'invention. Dans l'exemple du segment d'artère cible 1 illustré à la figure la, ce dernier présente un tronçon principal 5, formé de l'aorte thoracique puis de l'aorte abdominale, qui se termine à une extrémité distale par une fourche formant la bifurcation aortique. Au niveau du tronçon principal 5, le segment d'artère cible 1 comporte un certain nombre d'artères collatérales 2, dont les artères iliaques droite 3 et gauche 4 et deux artères supplémentaires sont ici seulement représentées en figure la, comme les artères iliaques communes, les artères iliaques internes, les artères iliaques externes, les artères rénales, l'artère mésentérique supérieure, le tronc coeliaque, les artères sous-clavières, la carotide primitive gauche. Le but de l'étape 105 du procédé 100 d'assistance à la réalisation d'un dispositif sur mesure déployable implantable dans une cavité d'un corps d'un être vivant selon l'invention est de reconstruire en trois dimensions une surface complexe 6 délimitant l'ensemble du segment d'artère cible 1 à instrumenter d'une endoprothèse sur mesure. Les techniques de reconstruction tridimensionnelle à partir d'une série d'images radiographiques sont connues en soi et ne seront pas développées plus avant ici. Cela permet d'obtenir un modèle numérique de la géométrie du segment d'artère cible 1. Par exemple, ici la reconstruction tridimensionnelle de la surface complexe 6 est effectuée à l'aide d'une technique de segmentation connue en soi, appliquée sur la série d'images radiographiques, de sorte à obtenir un ensemble de surfaces simples formant la surface complexe 6. De plus, lors du traitement d'images permettant la reconstruction tridimensionnelle du segment d'artère cible 1, des repères géométriques sont générés le long des artères collatérales 2, en particulier des points constituant une ligne centrale propre à chaque artère et des points constituant des contours du lumen artériel autour de chaque point constituant la ligne centrale propre à chaque artère comme cela est représenté en figure la.

Dans une étape suivante 110, le procédé 100 d'assistance à la réalisation d'un dispositif sur mesure déployable implantable dans une cavité d'un corps d'un être vivant selon l'invention réalise un premier maillage par éléments finis d'une géométrie de la surface complexe 6 déterminée et reconstruite lors de l'étape 105. Ce premier maillage est obtenu, par exemple, à partir de l'ensemble de surfaces simples formant la surface complexe 6, à l'aide d'un algorithme de maillage connu en soi dans le domaine des éléments finis. Le premier maillage par éléments finis est alors défini par un ensemble d'éléments coques, affectés chacun d'une épaisseur pouvant varier suivant la localisation de l'élément, d'une loi de comportement élastique anisotrope pouvant varier suivant la composition locale du tissu artériel qu'il représente, d'une orientation matériau variant le long des artères et définie à la fin de la déformation du maillage par la morphose. D'autre part, dans une sous-étape 111, le procédé 100 d'assistance à la réalisation d'un dispositif sur mesure déployable implantable dans une cavité d'un corps d'un être vivant selon l'invention détermine un ensemble de paramètres géométriques des points d'intérêt 2 que forment, ici, les artères collatérales 2, et en particulier leur ostias. Ces paramètres géométriques sont au moins la taille et la position des points d'intérêt 2. A cela, peut s'ajouter l'orientation au niveau de leur ostias des artères collatérales 2.

Lors d'une étape 115, le procédé 100 d'assistance à la réalisation d'un dispositif sur mesure déployable implantable dans une cavité d'un corps d'un être vivant selon l'invention permet de choisir un ou plusieurs composants génériques 230,240,250 d'un dispositif déployable 200 dont la personnalisation est à réaliser pour le rendre sur mesure au patient à opérer. Les composants génériques 230,240,250 sont formés d'un textile sous forme de surfaces simples comme des surfaces tubulaires de révolution et de stents sous forme de courbes tridimensionnelles simples, et sont éventuellement assemblés ensemble pour former, ici, une endoprothèse à personnaliser. Les composants génériques 230,240,250 peuvent provenir d'une bibliothèque d'endoprothèses génériques numérisées d'un ou plusieurs fabricants d'endoprothèses. En particulier, la bibliothèque peut contenir des corps principaux bifurqués, des jambages iliaques, des extensions proximales, des stents couverts, utilisées dans le cadre de la chirurgie endovasculaire des anévrismes des artères iliaques, de l'aorte abdominale ou de l'aorte thoracique. Ces endoprothèses étant génériques, elles ne présentent pas, à ce stade, d'éléments personnalisés telles que des fenêtres, des branches, etc...

Ensuite lors d'une étape 120, le procédé 100 d'assistance à la réalisation d'un dispositif sur mesure déployable implantable dans une cavité d'un corps d'un être vivant selon l'invention réalise un deuxième maillage par éléments finis du ou des composants génériques 230,240,250 choisis et éventuellement assemblés lors de l'étape 115. De nouveau, comme pour le premier maillage, le deuxième maillage est obtenu, par exemple, par un algorithme de maillage à partir d'un ensemble de surfaces ou de courbes simples issues du ou des composants génériques 230,240,250 choisis et éventuellement assemblés. Le deuxième maillage par éléments finis est alors défini par un ensemble d'éléments coques pour le textile et d'éléments poutres pour les stents, affectés d'une épaisseur pour le textile et d'un diamètre pour les stents dépendants des composants génériques choisis, d'une loi de comportement orthotrope élastique pour le textile et isotrope élastique pour les stents.

Il est à noter que les étapes 105 à 120 peuvent être réalisées dans n'importe quel ordre du moment que l'étape 110 survient après l'étape 105 et que, de manière similaire, l'étape 120 survient après l'étape 115.

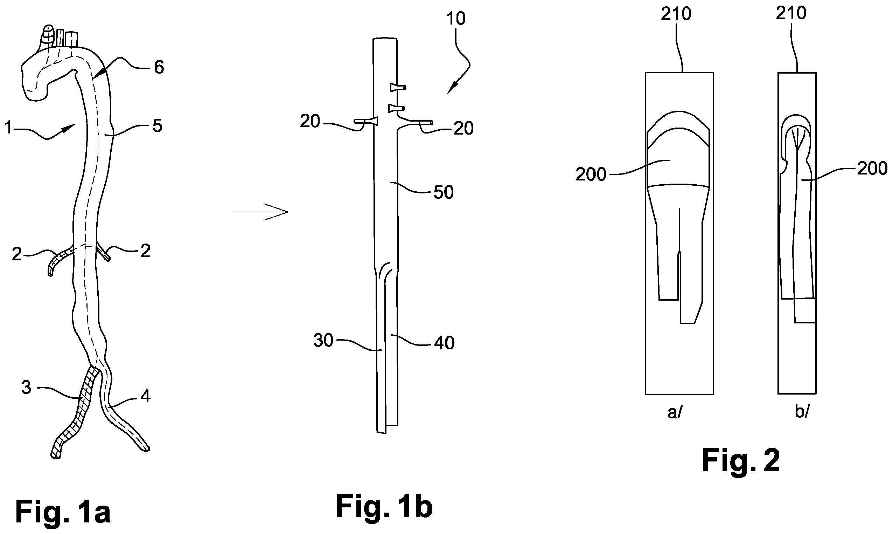

Une fois les premier et deuxième maillages par éléments finis réalisés, le procédé 100 d'assistance à la réalisation d'un dispositif sur mesure déployable implantable dans une cavité d'un corps d'un être vivant selon l'invention réalise une étape 125 lors de cette étape 125, le procédé 100 selon l'invention détermine des paramètres d'une morphose du premier maillage de la surface complexe 6 du segment d'artère cible 1 reconstruit vers le deuxième maillage du ou des composants génériques 230,240,250 choisis et éventuellement assemblés. Les paramètres de la morphose comprennent des trajectoires lisses de chacun des nœuds du premier maillage pour passer de la forme initiale illustrée en figure la vers la forme finale illustrée en figure 1b, trajectoires déterminées alors par le procédé 100 d'assistance à la réalisation d'un dispositif sur mesure déployable implantable dans une cavité d'un corps d'un être vivant selon l'invention. Une fois les paramètres de la morphose déterminés, il est possible de déformer, à partir des paramètres de la morphose, par simulation, le premier maillage de la surface complexe 6 du segment d'artère cible 1 vers un maillage d'une géométrie d'une surface d'un ensemble de volumes simples 20,30,40,50 raccordés les uns aux autres représentatifs du segment d'artère cible déformé 10. Cet ensemble de volumes simples 20,30,40,50 présente ainsi des dimensions qui sont alors ajustées à celles du ou des composants génériques 230,240,250 choisis et éventuellement assemblés pour former l'endoprothèse à personnaliser.

Ensuite, dans une étape 130 ultérieure, le procédé 100 d'assistance à la réalisation d'un dispositif sur mesure déployable implantable dans une cavité d'un corps d'un être vivant selon l'invention simule par la méthode des éléments finis une déformation par morphose inverse en fonction des paramètres de la morphose précédemment déterminés du deuxième maillage vers la géométrie de la surface complexe 6 de sorte à simuler un déploiement du dispositif sur mesure déployable 200 qu'est, ici, l'endoprothèse à personnaliser. Cette étape 130 permet de déterminer la façon dont l'endoprothèse sur mesure en cours d'élaboration va se déployer au sein du segment d'artère cible 1 du patient à opérer. En particulier, lors de cette morphose inverse, le procédé 100 d'assistance à la réalisation d'un dispositif sur mesure déployable implantable dans une cavité d'un corps d'un être vivant selon l'invention impose aux nœuds du deuxième maillage les trajectoires, en sens opposé, déterminées précédemment lors de la morphose de l'étape 125 précédente, jusqu'au déploiement du deuxième maillage.

Cette simulation peut s'effectuer avec le deuxième maillage préalablement inséré, dans une sous étape 132, dans le premier maillage de la surface complexe 6 de la cavité 1 que forme ici le segment d'artère cible. Ainsi une fois l'étape 130 effectuée, le deuxième maillage de l'endoprothèse à personnaliser est déployé par la morphose inverse qui vient d'être réalisée. En particulier, lors de cette morphose inverse, le procédé 100 d'assistance à la réalisation d'un dispositif sur mesure déployable implantable dans une cavité d'un corps d'un être vivant selon l'invention impose aux nœuds du deuxième maillage les trajectoires, en sens opposé, déterminées précédemment lors de la morphose de l'étape 125 précédente, jusqu'au déploiement du deuxième maillage à l'intérieur du premier maillage de la surface complexe 6, en utilisant un solveur par la méthode des éléments finis. Le deuxième maillage est alors en contact avec le premier maillage de la surface complexe 6. De là, dans une étape 134, le procédé 100 d'assistance à la réalisation d'un dispositif sur mesure déployable implantable dans une cavité d'un corps d'un être vivant selon l'invention calcule, toujours par la méthode des éléments finis des déformations et contraintes induites par les interactions entre les premier et deuxième maillages.

De manière optionnelle, le procédé 100 d'assistance à la réalisation d'un dispositif sur mesure déployable implantable dans une cavité d'un corps d'un être vivant selon l'invention, dans une étape 131 préliminaire à l'étape 130, fait subir au deuxième maillage par la méthode des éléments finis une déformation simulant une compression de l'endoprothèse 200 à élaborer à l'intérieur d'une gaine d'implantation. Cela permet d'approcher de manière optimale les conditions d'implantation dans le patient à opérer de l'endoprothèse 200 sur mesure déployable finale. De plus, cela permet de simuler une introduction de ce deuxième maillage comprimé dans le premier maillage selon une position proche d'une position optimale afin de parfaire l'implantation réelle ultérieure sur le patient à opérer. D'autre part, il est possible de simuler ainsi plusieurs positions de déploiement de l'endoprothèse 200 proche de la position optimale ou initiale visée pour évaluer l'impact d'un positionnement imprécis de l'endoprothèse 200 durant l'intervention réelle ultérieure sur le patient.

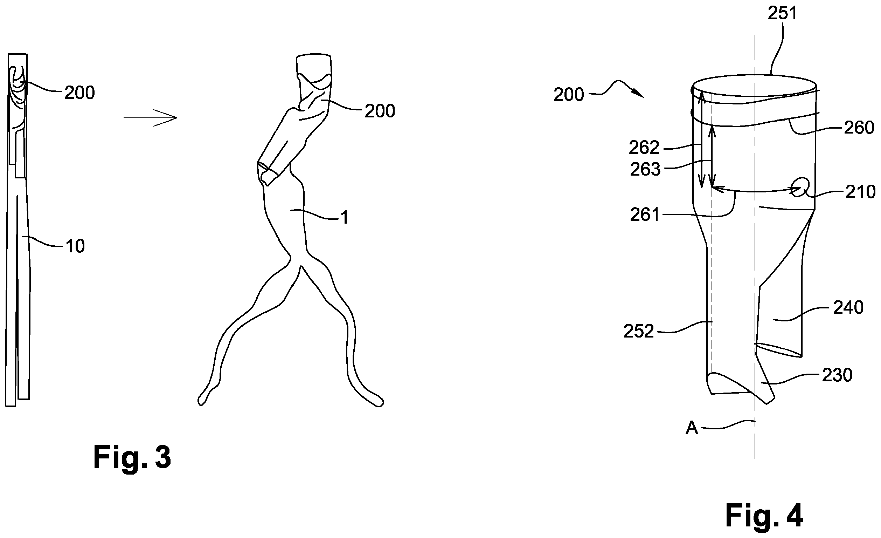

Dès lors, le procédé 100 d'assistance à la réalisation d'un dispositif sur mesure déployable implantable dans une cavité d'un corps d'un être vivant selon l'invention réalise une étape 135 durant laquelle va être déterminé un dimensionnement de points d'intérêt 2 du segment d'artère cible 1 présents sur le premier maillage de la surface complexe 6 et qui se retrouvent en regard du deuxième maillage déployée de l'endoprothèse à réaliser. En particulier, dans le cas de l'endoprothèse 200 à personnaliser, les points d'intérêt sont les ostias des artères collatérales 2. Cela se traduit sur l'endoprothèse 200 à personnaliser par le repérage et la réalisation de fenêtres 210 dans une paroi de l'endoprothèse 200, fenêtres 210 qui suite au déploiement dans le segment d'artère cible 1 se retrouveront en regard des ostias précités. En pratique, comme illustré par exemple en figure 4, cela consiste à déterminer des coordonnées des fenêtres 210 par rapport à des points de repères 251,260 et à un plan de référence défini par exemple par le plan passant par un axe longitudinal A de l'endoprothèse 200 générique à personnaliser et une génératrice 252 de celle-ci. Par exemple, les points de repère peuvent être :
- le bord 251 de l'extrémité supérieure de l'endoprothèse 200 associé alors avec une première latitude 262 ; et/ou
- une référence 260 propre au fabriquant de l'endoprothèse 200 générique à personnaliser associée à une deuxième latitude 263.

Associée au plan de référence, une longitude 261 est définie soit par une distance circonférentielle soit par un angle. En variante, le plan de référence peut être défini par rapport au patient qui va recevoir l'endoprothèse 200 sur mesure en cours d'élaboration.

Afin de déterminer les coordonnées et taille des points d'intérêt 2, le procédé 100 d'assistance à la réalisation d'un dispositif sur mesure déployable implantable dans une cavité d'un corps d'un être vivant selon l'invention réalise une projection de ceux-ci à partir du premier maillage de la surface complexe 6 sur le deuxième maillage déformé lors du déploiement réalisé à l'étape 130. La projection s'effectue suivant l'axe des artères collatérales 2 sur le deuxième maillage déformé par la morphose inverse de l'étape 130. En particulier, cette projection consiste à déterminer des nœuds du deuxième maillage qui se trouvent en regard des points d'intérêt 2.

Une fois l'étape 135 réalisé, le procédé 100 d'assistance à la réalisation d'un dispositif sur mesure déployable implantable dans une cavité d'un corps d'un être vivant selon l'invention génère, dans une étape 140 un ou plusieurs plans cotés de l'endoprothèse 200 sur mesure déployable, en particulier du ou des composants génériques formant l'endoprothèse 200 sur lesquels a été transposé le dimensionnement, c'est-à-dire les taille et position des points d'intérêt 2 précédemment déterminé à l'étape 135. En particulier, la transposition du dimensionnement est réalisée à partir des coordonnées des nœuds du deuxième maillage précédemment déterminés dans le deuxième maillage non déformé. Ces plans cotés permettent par la suite la réalisation 101 effective de l'endoprothèse 200 sur mesure déployable adaptée au patient qui doit la recevoir.

Dans une étape 145 additionnelle, le procédé 100 d'assistance à la réalisation d'un dispositif sur mesure déployable implantable dans une cavité d'un corps d'un être vivant selon l'invention réalise une extraction d'un ensemble d'indicateurs permettant à un praticien de déterminer la pertinence du planning opératoire à appliquer au patient à opérer pour lequel l'endoprothèse 200 sur mesure déployable a été personnalisée lors des étapes précédentes. Cela lui permet d'étudier l'influence des paramètres d'interventions, telle que la position de largage au sein du segment d'artère cible 1 de l'endoprothèse 200, pour étudier l'impact potentiel d'imprécisions de déploiement de l'endoprothèse 200 lors de l'intervention réelle ultérieure.

Le procédé 100 d'assistance à la réalisation d'un dispositif sur mesure déployable implantable dans une cavité d'un corps d'un être vivant selon l'invention a été décrit en regard d'une application à des endoprothèses vasculaires sur mesures destinées à être implantées dans un lumen artériel. Toutefois, le procédé 100 d'assistance à la réalisation d'un dispositif sur mesure déployable implantable dans une cavité d'un corps d'un être vivant selon l'invention peut être mise en œuvre pour tout type de dispositifs, déployables ou non, sur mesure implantables dans une cavité quelconque du corps d'un être vivant.

D'autre part, le procédé 100 d'assistance à la réalisation d'un dispositif sur mesure déployable implantable dans une cavité d'un corps d'un être vivant selon l'invention est mise en œuvre par un programme de commande d'un calculateur, comme un ordinateur. Pour cela, le programme de commande est enregistré et stocké sur un support informatique compatible avec le calculateur. Il peut être prévu des phases de visualisation d'un résultat des différentes étapes du procédé 100 d'assistance à la réalisation d'un dispositif sur mesure déployable implantable dans une cavité d'un corps d'un être vivant selon l'invention, soit en fin d'étape soit en cours d'étape.

Bien entendu, il est possible d'apporter à l'invention de nombreuses modifications sans pour autant sortir du cadre de celle-ci.

## Revendications

1. Procédé (100) d'assistance à la réalisation d'un dispositif sur mesure déployable implantable dans une cavité d'un corps d'un être vivant à partir d'une série d'images radiographiques de la cavité, le procédé comportant des étapes de :
a. Reconstruction (105) tridimensionnelle d'une surface complexe délimitant une partie de la cavité dans laquelle le dispositif sur mesure déployable est destiné à être mis en place ;
b. Réalisation (110) d'un premier maillage par éléments finis d'une géométrie de la surface complexe ainsi reconstruite ;
c. Choix (115) d'un ou plusieurs composants génériques du dispositif sur mesure déployable dont la personnalisation est à réaliser ;
d. Réalisation (120) d'un deuxième maillage par éléments finis du ou des composants génériques choisis et assemblés pour former le dispositif sur mesure déployable ;
e. Détermination (125) des paramètres d'une morphose du premier maillage obtenu vers un maillage d'une géométrie formée d'un ensemble de volumes simples raccordés les uns aux autres représentatif de la partie de la cavité, puis déformation du premier maillage par cette morphose;
f. Simulation (130) par la méthode des éléments finis de la déformation du deuxième maillage ainsi obtenu, par morphose inverse en fonction des paramètres de la morphose précédemment déterminés, vers la géométrie de la surface complexe de la cavité de sorte à simuler un déploiement du dispositif sur mesure déployable une fois mis en place dans la cavité ; **caractérisé en ce que** procédé comporte
g. Détermination (135) d'un dimensionnement de points d'intérêt de la partie considérée de la cavité par projection de ceux-ci sur le deuxième maillage ainsi déformé ; et
h. Génération (140) d'un plan coté du ou des composants génériques sur lesquels le dimensionnement précédemment déterminé a été transposé, le plan coté permettant la réalisation du dispositif sur mesure déployable.

2. Procédé selon la revendication 1, **caractérisé en ce que**, lors de l'étape b, le procédé comporte une sous étape de détermination (111) de paramètres géométriques de points d'intérêt de la partie considérée de la cavité, les paramètres géométriques étant utilisés lors de l'étape g.

3. Procédé selon la revendication 1 ou 2, **caractérisé en ce que**, suite à l'étape e et préalablement à l'étape f, le deuxième maillage subit une déformation (131) simulant une compression du dispositif sur mesure déployable assemblé à l'intérieur d'une gaine d'implantation.

4. Procédé selon l'une des revendications 1 à 3, **caractérisé en ce que**, lors de l'étape f, le procédé comporte une étape préliminaire d'insertion (132) du deuxième maillage dans le premier maillage déformé.

5. Procédé selon la revendication 4, **caractérisé en ce que**, suite à l'étape f et préalablement à l'étape g, le procédé comporte une étape de calcul (134) par simulation par la méthode des éléments finis de déformations et contraintes induites par des interactions entre les premier et deuxième maillages suite à la déformation par morphose inverse du deuxième maillage vers le premier maillage.

6. Procédé selon l'une des revendications 1 à 5, **caractérisé en ce que**, lors de l'étape g, la projection consiste à déterminer les nœuds du deuxième maillage en regard des points d'intérêt.

7. Procédé selon la revendication 6, **caractérisé en ce que**, lors de l'étape h, la transposition du dimensionnement s'effectue à partir des coordonnées des nœuds précédemment déterminés lors de l'étape g dans le deuxième maillage non déformé.

8. Procédé selon la revendication 3, **caractérisé en ce que**, suite à la déformation simulant une compression, le deuxième maillage ainsi déformé subit une simulation d'introduction dans le premier maillage selon une position proche d'une position optimale.

9. Procédé selon l'une des revendications 1 à 8, **caractérisé en ce qu'**il comporte une étape supplémentaire de
i. Extraction (145) d'un ensemble d'indicateurs permettant à un praticien de déterminer la pertinence d'un planning préopératoire.

10. Procédé selon l'une des revendications 1 à 9, **caractérisé en ce que**, la cavité étant un lumen artériel, le dispositif sur mesure déployable est une endoprothèse aortique.

11. Support informatique comportant l'enregistrement d'un programme de commande d'un calculateur, pour la mise en œuvre du procédé conforme à l'une au moins des revendications 1 à 10.

## Patentansprüche

1. Verfahren (100) zur Unterstützung der Herstellung einer entfaltbaren maßgefertigten Vorrichtung, die in einen Hohlraum eines Körpers eines Lebewesens implantierbar ist, anhand einer Reihe von Röntgenbildern des Hohlraums, wobei das Verfahren die Schritte umfasst:
a. dreidimensionale Rekonstruktion (105) einer komplexen Fläche, die einen Teil des Hohlraums begrenzt, in welchem die entfaltbare maßgefertigte Vorrichtung platziert werden soll;
b. Herstellung (110) eines ersten Finite-Elemente-Gitters einer Geometrie der so rekonstruierten komplexen Fläche;
c. Wahl (115) einer oder mehrerer generischer Komponenten der entfaltbaren maßgefertigten Vorrichtung, deren Individualisierung durchzuführen ist;
d. Herstellung (120) eines zweiten Finite-Elemente-Gitters der generischen Komponente oder Komponenten, die gewählt und zusammengesetzt wurden, um die entfaltbare maßgefertigte Vorrichtung zu bilden;
e. Bestimmung (125) der Parameter eines Morphings vom erhaltenen ersten Gitter zu einem Gitter einer von einer Menge von aneinander angeschlossenen einfachen Volumina gebildeten Geometrie, das für den Teil des Hohlraums repräsentativ ist, anschließend Verformung des ersten Gitters durch dieses Morphing;
f. Simulation (130), durch die Finite-Elemente-Methode, der Verformung des so erhaltenen zweiten Gitters durch inverses Morphing, in Abhängigkeit von den zuvor bestimmten Parametern des Morphings, zur Geometrie der komplexen Fläche des Hohlraums, um eine Entfaltung der entfaltbaren maßgefertigten Vorrichtung, nachdem sie in dem Hohlraum platziert worden ist, zu simulieren;
**dadurch gekennzeichnet, dass** das Verfahren umfasst:
g. Bestimmung (135) einer Bemessung von Punkten von Interesse des betrachteten Teils des Hohlraums durch Projektion derselben auf das so verformte zweite Gitter; und
h. Erzeugung (140) einer Maßzeichnung der generischen Komponente oder Komponenten, auf welche die zuvor bestimmte Bemessung übertragen worden ist, wobei die Maßzeichnung die Herstellung der entfaltbaren maßgefertigten Vorrichtung ermöglicht.

2. Verfahren nach Anspruch 1, **dadurch gekennzeichnet, dass** in Schritt b das Verfahren einen Teilschritt der Bestimmung (111) geometrischer Parameter von Punkten von Interesse des betrachteten Teils des Hohlraums umfasst, wobei die geometrischen Parameter in Schritt g verwendet werden.

3. Verfahren nach Anspruch 1 oder 2, **dadurch gekennzeichnet, dass** im Anschluss an Schritt e und vor Schritt f das zweite Gitter eine Verformung (131) erfährt, die ein Zusammendrücken der zusammengebauten entfaltbaren maßgefertigten Vorrichtung im Inneren einer Implantationshülse simuliert.

4. Verfahren nach einem der Ansprüche 1 bis 3, **dadurch gekennzeichnet, dass** in Schritt f das Verfahren einen vorausgehenden Schritt des Einfügens (132) des zweiten Gitters in das verformte erste Gitter umfasst.

5. Verfahren nach Anspruch 4, **dadurch gekennzeichnet, dass** im Anschluss an Schritt f und vor Schritt g das Verfahren einen Schritt der Berechnung (134), durch Simulation durch die Finite-Elemente-Methode, von Verformungen und Beanspruchungen umfasst, die durch Interaktionen zwischen dem ersten und dem zweiten Gitter im Anschluss an die Verformung durch inverses Morphing vom zweiten Gitter zum ersten Gitter hervorgerufen werden.

6. Verfahren nach einem der Ansprüche 1 bis 5, **dadurch gekennzeichnet, dass** in Schritt g die Projektion darin besteht, die Knoten des zweiten Gitters gegenüber den Punkten von Interesse zu bestimmen.

7. Verfahren nach Anspruch 6, **dadurch gekennzeichnet, dass** in Schritt h die Übertragung der Bemessung ausgehend von den Koordinaten der Knoten erfolgt, die zuvor in Schritt g in dem nicht verformten zweiten Gitter bestimmt wurden.

8. Verfahren nach Anspruch 3, **dadurch gekennzeichnet, dass** im Anschluss an die ein Zusammendrücken simulierende Verformung das so verformte zweite Gitter einer Simulation der Einführung in das erste Gitter an einer Position, die einer optimalen Position nahekommt, unterzogen wird.

9. Verfahren nach einem der Ansprüche 1 bis 8, **dadurch gekennzeichnet, dass** es einen zusätzlichen Schritt umfasst:
i. Extraktion (145) einer Menge von Indikatoren, die einem Mediziner ermöglichen, die Richtigkeit einer Operationsplanung zu bestimmen.

10. Verfahren nach einem der Ansprüche 1 bis 9, **dadurch gekennzeichnet, dass**, wenn der Hohlraum ein arterielles Lumen ist, die entfaltbare maßgefertigte Vorrichtung eine Aortenendoprothese ist.

11. Datenträger, welcher die Aufzeichnung eines Programms zur Steuerung eines Computers für die Ausführung des Verfahrens gemäß wenigstens einem der Ansprüche 1 bis 10 umfasst.

## Claims

1. A method (100) for assisting in the production of an implantable unfurlable made-to-measure device that can be implanted in a cavity of a body of a living being, based on a series of radiographic images of the cavity, the method comprising steps of:
a. Three-dimensional reconstruction (105) of a complex surface delimiting a part of the cavity in which the unfurlable made-to-measure device is intended to be installed;
b. Production (110) of a first finite element mesh of a geometry of the complex surface thus reconstructed;
c. Choosing (115) of one or more generic components of the unfurlable made-to-measure device that is to be personalized;
d. Production (120) of a second finite element mesh of the generic component or components chosen and assembled to form the unfurlable made-to-measure device;
e. Determination (125) of the parameters of a morphing of the obtained first mesh to a mesh of a geometry formed from a set of simple volumes linked to each other representative of the part of the cavity, and then deformation of the first mesh by this morphing;
f. Simulation (130), by the finite elements method, of the deformation of the second mesh thus obtained, by inverse morphing, dependent on the previously determined morphing parameters, to the geometry of the complex surface of the cavity, so as to simulate the unfurling of the unfurlable made-to-measure device once installed in the cavity;
**characterized in that** the method comprises:
g. Determination (135) of a dimensioning of points of interest of the part in question of the cavity by projecting them onto the second mesh thus deformed; and
h. Generation (140) of a dimensioned drawing of the generic component or components onto which the previously determined dimensioning has been transposed, the dimensioned drawing allowing the unfurlable made-to-measure device to be produced.

2. The method as claimed in claim 1, **characterized in that**, during step b, the method comprises a sub-step of determining (111) geometric parameters of points of interest of the part in question of the cavity, the geometric parameters being used during step g.

3. The method as claimed in claim 1 or 2, **characterized in that**, following step e and prior to step f, the second mesh undergoes a deformation (131) simulating a compression of the assembled unfurlable made-to-measure device inside a delivery sheath.

4. The method as claimed in one of claims 1 to 3, **characterized in that**, during step f, the method comprises a preliminary step of inserting (132) the second mesh into the deformed first mesh.

5. The method as claimed in claim 4, **characterized in that**, following step f and prior to step g, the method comprises a step of calculating (134), by simulation by the finite elements method, deformations and constraints induced by interactions between the first and second meshes following the deformation by inverse morphing of the second mesh to the first mesh.

6. The method as claimed in one of claims 1 to 5, **characterized in that**, during step g, the projection consists in determining the nodes of the second mesh facing the points of interest.

7. The method as claimed in claim 6, **characterized in that**, during step h, the transposing of the dimensioning is carried out based on the coordinates of the nodes previously determined during step g in the non-deformed second mesh.

8. The method as claimed in claim 3, **characterized in that**, following the deformation simulating a compression, the deformed second mesh undergoes a simulation of insertion into the first mesh into a position close to an optimal position.

9. The method as claimed in one of claims 1 to 8, **characterized in that** it comprises an additional step of
i. Extracting (145) a set of indicators enabling a practitioner to determine the suitability of a preoperative plan.

10. The method as claimed in one of claims 1 to 9, **characterized in that**, the cavity being an arterial lumen, the unfurlable made-to-measure device is an aortic endoprosthesis.

11. A computer medium comprising the recording of a program for controlling a calculator, for implementing the method according to at least one of claims 1 to 10.
